# NEW EUROPEAN PATENT SPECIFICATION

(11) **EP 1 563 091 B2**
(45) Date of publication and mention of the opposition decision: **27.06.2012**
(45) Mention of the grant of the patent: 07.01.2009
(21) Application number: 03770642.1
(22) Date of filing: 03.10.2003
(51) Int. Cl.: C12N 15/10, C12Q 1/68

(54) **METHODS AND MATERIALS FOR USING CHEMICAL COMPOUNDS AS A TOOL FOR NUCLEIC ACID STORAGE ON MEDIA OF NUCLEIC ACID PURIFICATION SYSTEMS**
VERFAHREN UND MATERIALIEN ZUR VERWENDUNG CHEMISCHER VERBINDUNGEN ALS HILFSMITTEL ZUR NUKLEINSÄURELAGERUNG AUF MEDIEN VON NUKLEINSÄUREREINIGUNGSSYSTEMEN
METHODES ET MATIERES POUR UNE UTILISATION DE COMPOSES CHIMIQUES, EN TANT QU'OUTILS DE STOCKAGE D'ACIDE NUCLEIQUE SUR UN SUPPORT DE SYSTEMES DE PURIFICATION D'ACIDE NUCLEIQUE

(30) Priority: 04.10.2002 US 416356 P
(43) Date of publication of application: 17.08.2005
(73) Proprietor: GE Healthcare Bio-Sciences Corp., Piscataway, N.J. 08855 (US)
(72) Inventor: SMITH, Martin, A., Montclair, NJ 07043 (US); FOMOVSKAIA, Galina, N., Port Washington, NY 11050 (US); FOMOVSKY, Mikhail, A., Port Washington, NY 11050 (US)
(74) Representative: Bryan, Ian Bennett
(86) International application number: PCT/US2003/031483
(87) International publication number: WO 2004/033470

(56) References cited:
- WO-A-00/21973
- WO-A-00/53807
- WO-A-00/62023
- WO-A-90/03959
- WO-A-02/072870
- WO-A1-96/39813
- WO-A1-99/39009
- WO-A2-02/40699
- WO-A2-96/18731
- WO-A2-99/38962
- WO-A2-02/040699
- US-A- 5 496 562
- US-A- 5 756 126
- US-A- 5 807 527

## Description

### Field of the Invention

The present invention relates to methods for storing nucleic acid from a sample containing nucleic acid, such as a cell sample or cell lysate. The nucleic acid is isolated and can be stored efficiently, for extended periods of time at room temperature and humidity, on a wide variety of filters and other types of solid phase media. The invention provides methods for storing nucleic acid-containing samples on a wide range of solid phase media in many types of tubes, columns, or multiwell plates, many of which are commercial available.

### Background of the Invention

Genotyping is the discipline of identifying an individual's genome in relation to disease specific alleles and/or mutations that occur as an effect of parental linkage. The rapid purification of human genomic DNA is an essential part of a genotyping process; the genomic DNA of an individual being the structural unit for the entire DNA sequence of every allele expressed.

Sequencing human DNA is a complex operation. In order to carry out sequence analysis on regions of the chromosomes that may contain portions of mutation or disease specific sequences, selected portions are amplified, e.g., via PCR, and the amplified products are sequenced. The selected portions of the chromosomes that are amplified are dictated by the specific sequence of the primers used in the PCR amplification, such as those used for linkage studies to determine that a disease-bearing sequence is on a particular chromosome. The primer sets that are used in genotyping studies are commercially available and are representative for the chromosome under examination. Due to the large length of chromosomes, many PCR reactions are carried out on the genomic DNA template from a single patient.

While relatively rapid and convenient procedures for the purification of various types nucleic acid (such as genomic DNA, complementary DNA (DNA), mitochondrial or chloroplast DNA, or various types of RNA) from agarose have been developed, it remains a relatively difficult operation to extract nucleic acid directly from more complex starting samples such as cells and cell lysates. On the whole, most procedures currently practiced to purify nucleic acid from nucleic acid-containing samples comprising cells or cell lysates remain time consuming and labor intensive. In addition, storage of the isolated nucleic acid usually entails maintaining a vial of the nucleic acid in solution in a refrigerator or, preferably, in a freezer. Long-term storage of numerous samples requires a large amount of dedicated freezer space. These storage requirements result in high energy consumption and expense and render nucleic acid isolation in the field difficult or even in some circumstances impossible.

There have been attempts to minimize the laborious and time-consuming steps of the previously used methods for isolating nucleic acid from these more complex samples. One such method is described in EP 0389063. The method disclosed in EP 0389063 involves mixing the cell sample (such as whole blood) with a chaotropic substance and a particulate nucleic acid binding solid phase comprising silica or a derivative thereof. It is known that, in the presence of a chaotropic substance, nucleic acid is released from cells and binds to silica-based nucleic acid binding solid phases. Subsequently, the mixture is centrifuged to pellet the solid phase with the nucleic acid bound thereto and the supernatant is discarded. The pelleted material is subjected to several washing steps with the chaotropic agent and organic solvents. Finally, the DNA is eluted from the solid phase in a low salt buffer.

The method described in EP 0389063 is disadvantageous in that it is a manually intensive, multi-step procedure. In view of the fact that the method involves a number of centrifugation and vessel transfer steps, this method is unsuitable for automation. In addition, the eluted DNA would still require storage in solution at low temperature.

U.S. Patents Nos. 5,187,083 and 5,234,824 each describe a method for rapidly obtaining substantially pure DNA from a biological sample containing cells. The methods involve gently lysing the membranes of the cells to yield a lysate containing genomic DNA in a high molecular weight form. The lysate is moved through a porous filter to trap selectively the high molecular weight DNA on the filter. The DNA is released from the filter using an aqueous solution.

There have been attempts to facilitate purification of human genomic DNA using a variety of methods (Molecular Cloning, Sambrook et al. (1989)). Consequently, many commercial kit manufacturers provide products for such techniques, for example: AmpReady^{™} (Promega, Madison, Wisconsin), DNeasy™ (Qiagen, Valencia, California), and Split Second^{™} (Roche Molecular Biochemicals, Indianapolis, Indiana). These products rely on the use of specialized matrices or buffer systems for the rapid isolation of the genomic DNA molecule.

More recently, there have been attempts to use microporous filter-based techniques as tools for the purification of genomic DNA as well as a whole multitude of nucleic acids. The advantage of filter-based matrices are that they can be fashioned into many formats that include tubes, spin tubes, sheets, and microwell plates. Microporous filter membranes as purification support matrices have other advantages within the art. They provide a compact, easy to manipulate system allowing for the capture of the desired molecule and the removal of unwanted components in a fluid phase at higher throughput and faster processing times than possible with column chromatography. This is due to the fast diffusion rates possible on filter membranes.

Nucleic acid molecules have been captured on filter membranes, generally either through simple adsorption or through a chemical reaction between complementary reactive groups present on the filter membrane or on a filter-bound ligand resulting in the formation of a covalent bond between the ligand and the desired nucleic acid.

Porous filter membrane materials used for non-covalent nucleic acid immobilization have included materials such as nylon, nitrocellulose, hydrophobic polyvinylidinefluoride (PVDF), and glass microfiber. A number of methods and reagents have also been developed to allow the direct coupling of nucleic acids onto solid supports, such as oligonucleotides and primers. UV cross-linking of DNA (Church et al., PNAS, vol. 81, page 1991, 1984), The Generation Capture Column Kit (Gentra Systems, Minneapolis, MN) and RNA to nylon membranes have also been reported.

Many chemical methods have been utilized for the immobilization of molecules such as nucleic acids on filter membranes. For example, activated paper (TransBind™, Schleicher & Schuell Ltd., Keene, N.H.), carbodimidazole-activated hydrogel-coated PVDF membrane (Immobilin-IAV™, Millipore Corp., Bedford, Mass.), MAP paper (Amersham, Littlechalfont Bucks, Wisconsin), activated nylon (BioDyne™, Pall Corp., (Glen Cove, N.Y.), DVS- and cyanogen bromide-activated nitrocellulose. Membranes bound with specific ligands are also known such as the SAM2™ Biotin Capture Membrane (Promega) which binds biotinylated molecules based on their affinity to streptavidin or MAC affinity membrane system (protein A/G) (Amicon, Bedford, Massachusetts). Some of the disadvantages of covalent attachment of biomolecules onto activated membranes are:
a) Molecule immobilization is often slow, requiring 20-180 minutes for reaction completion.
b) High ligand and biomolecule concentration is needed for fast immobilization.
c) Constant agitation is needed during the immobilization process, which may result in biomolecule denaturation and deactivation.
d) Once the immobilization process is complete, often a blocking (capping) step is required to remove residual covalent binding capacity.
e) Covalently bound molecules cannot be retrieved from the filter membrane.

There is a need in various specific areas, such as forensics, for a nucleic acid immobilization media and procedure that exhibits the high specificity of covalent immobilization onto the filter membrane without the use of harsh chemical reactions and long incubation times, which can also be used at crime scenes, with blood sample archiving and other related uses. In particular there is a need for the capture and separation of nucleic acids from a mixture in a fluid phase onto a filter membrane matrix in forensics.

Of special interest is the ability to store or archive the bound nucleic acids on the filter membrane matrix for various uses. Alternatively, filters that permit elution of nucleic acids have uses in application requiring liquid formats. Embodiments of both types are found in the present invention.

Based on United States Patents 5,496,562, 5,756,126, and 5,807,527, it has been demonstrated that nucleic acids or genetic material can be immobilized to a cellulosic-based dry solid support or filter (FTA® filter). The solid support described is conditioned with a chemical composition that is capable of carrying out several functions: (i) lyse intact cellular material upon contact, releasing genetic material, (ii) enable and allow for the conditions that facilitate genetic material immobilization to the solid support (probably by a combination of mechanical and chaotrophic), (iii) maintain the immobilized genetic material in a stable state without damage due to degradation, endonuclease activity, UV interference, and microbial attack, and (iv) maintain the genetic material as a support-bound molecule that is not removed from the solid support during any down stream processing (as demonstrated by Del Rio et al (1995) BioTechniques. vol. 20: 970-974).

FTA® filters permit stable, long-term storage of nucleic acids, such as on a FTA® Card for archiving of DNA samples at room temperature for extended periods. The usefulness of the so called FTA® cellulosic filter material described in US patents 5,496,562, 5,756,126, and 5,807,527 has been illustrated for several nucleic acid techniques such as bacterial ribotyping (Rogers, C. & Burgoyne, L. (1997) Anal. Biochem., vol. 247: 223-227), detection of single base differences in viral and human DNA (Ibrahim et al. (1998) Anal. Chem., vol. 70: 2013-2017), DNA databasing (Ledray et al. (1997) J. Emergency Nursing., vol. 23, No. 2: 156-158), automated processing for STR electrophoresis (Belgrader, B. & Marino, M. (1996) L.R.A., vol. 9: 3-7, Belgrader et al. (1995) BioTechniques., vol. 19, No. 3: 427-432), and oligonucleotide ligation assay for diagnostics (Baron et al. (1996) Nature Biotech., vol. 14: 1279-1282).

More recently, glass microfiber, has been shown to specifically bind nucleic acids from a variety of nucleic acid containing sources very effectively (for example, see Itoh et al (1997) Simple and rapid preparation of plasmid template by filtration method using microtiter filter plates. NAR, vol. 25, No. 6: 1315-1316; Andersson, B. et al. (1996) Method for 96-well M13 DNA template preparations for large-scale sequencing: BioTechniques vol. 20: 1022-1027). Under the correct salt and buffering conditions, nucleic acids will bind to glass or silica with high specificity. A glass microfiber matrix in the form of a filter, possibly including a binder or a coating, such as FTA®, may be used for isolation and elution of nucleic acid.

Alternatively, silica, silica gel, glass particles, or glass mixtures not in the form of a filter may be suspended in a suspension and packed into a column or spin tube. Column packing material may also be obtained by the homogenization of glass fiber or microfiber filters having removal binders, followed by suspension as a "sludge," "resin," or "slurry" (see, e.g., U.S. Patent No., 5, 658,548).

It is well-known in the art that these types of columns, like protein fractionation columns, must be kept wet and not permitted to dry and that nucleic acids and proteins trapped in dried columns often cannot be eluted. This limitation necessitates the careful storage of packed columns under conditions to prevent drying (e.g., sealing to prevent exposure to air and possibly storage in a refrigerator or cold room). It also results in the need to conduct an isolation experiment within a relatively immediate time frame, during which careful and constant vigilance must be maintained to avoid drying of the column, either at the ends or internally due to air bubbles. Lengthy isolations may require the work to be performed in a cold room with frequent addition of buffer over the course of many hours.

WO-A-00/53807 describes a method for storing genetic material wherein the genetic material is immobilized on a support which has been pretreated with a chemical composition, wherein the support and coating function together to immobilize and stabilize the genetic material thereon.

It would be useful to provide methods for the storage of samples comprising nucleic acid with various types of media in a column, whereby the column with the sample captured on the media is dried and stored for days, weeks, or months at ambient temperature and humidity, followed by the isolation of the nucleic acids from the sample.

### Summary of the Invention

In one aspect, the present invention provides methods for isolating and storing nucleic acid, comprising:
a. providing a solid phase medium;
b. applying a sample comprising cells containing nucleic acid to the solid phase medium ;
c. retaining the cells with the solid phase medium as a cellular retentate and removing contaminants;
d. contacting the cellular retentate with a solution comprising a surfactant or detergent;
e. lysing the cellular retentate to form a cell lysate while retaining the cell lysate in the medium, the cell lysate comprising the nucleic acid;
f. drying the solid phase medium with the cell lysate comprising the nucleic acid; and
g. storing the dried solid phase medium with the nucleic acid.
h. eluting the nucleic acid from said medium, wherein the storage of the nucleic acid in step g has a duration of at least one week.

In a further aspect, the present invention provides methods for isolating and storing nucleic acid, comprising:
a. providing a solid phase medium;
b. applying a sample comprising cells containing nucleic acid to the solid phase medium;
c. retaining the cells with the solid phase medium as a cellular retentate and removing contaminants;
d. contacting the cellular retentate with a solution comprising:
   i. a weak base;
   ii. a chelating agent ; and
   iii. an anionic surfactant or detergent;
e. lysing the cellular retentate to form a cell lysate while retaining the cell lysate in the medium, the cell lysate comprising the nucleic acid;
f. drying the solid phase medium with the cell lysate comprising the nucleic acid;
g. storing the dried solid phase medium with the nucleic acid; and
h. eluting the nucleic acid from said medium, wherein the storage of the nucleic acid in step g has a duration of at least one week.

In one embodiment, the solution of step d further comprises uric acid or a urate salt.

In yet another aspect, the present invention provides methods for isolating and storing DNA, comprising:
a. providing a solid phase medium, wherein the solid phase medium comprises a filter comprising a plurality of fibers, wherein the fibers comprise:
   i. glass or silica-based fibers;
   ii. plastics-based fibers; or
   iii. nitrocellulose or cellulose-based fibers;
b. applying a sample comprising cells containing DNA to the solid phase medium;
c. retaining the cells with the solid phase medium as a cellular retentate and removing contaminants;
d. contacting the cellular retentate with a solution comprising:
   i. a weak base;
   ii. a chelating agent; and
   iii. an anionic surfactant or detergent;
e. lysing the cellular retentate to form a cell lysate while retaining the cell lysate in the medium, the cell lysate containing DNA;
f. drying the solid phase medium with the cell lysate comprising the DNA;
g. storing the dried solid phase medium with the DNA at a temperature of 5°C to 40°C;
h. heating the DNA with the solid phase medium to an elevated temperature of 65°C to 125°C; and
i. eluting the nucleic acid from said medium, wherein the storage of the nucleic acid in step g has a duration of at least one week.

In one embodiment, the solution of step d further comprises uric acid or a urate salt.

### Brief Description of the Drawings

**Figure 1A** is an agarose gel electrophoresis analysis of the quality of DNA extracted from frozen human whole blood and recovered from glass fiber media after 1 day storage at room temperature and humidity (M = molecular weight standard).
**Figure 1B** is an agarose gel electrophoresis analysis of the quality of DNA extracted from frozen human whole blood and recovered from glass fiber media after 5 days storage at room temperature and humidity (M = molecular weight standard).
**Figure 2** is an agarose gel electrophoresis analysis of the quality of DNA extracted from fresh human whole blood and recovered from glass fiber media after 20 days storage at room temperature and humidity (M = molecular weight standard).
**Figure 3** is an agarose gel electrophoresis analysis of the quality of DNA extracted from fresh human whole blood and recovered from glass fiber media after 3.5 months storage at room temperature and humidity (M = molecular weight standard).
**Figure 4** is an agarose gel electrophoresis analysis of the quality of the DNA isolated after one month storage at room temperature on the glass fiber GF/L-6^{™} and DBS^{™} media (Whatman), control vs. FTA treated (M = molecular weight standard).
**Figure 5** is an agarose gel electrophoresis analysis of the quality of the DNA isolated after three and one-half months storage at room temperature on the glass fiber GF/L^{™} GenFast column media (Whatman), control (bottom) vs. FTA® treated (top) (MW = molecular weight standard.
**Figure 6** is an agarose gel electrophoresis analysis of the quality of the DNA isolated after five months storage at room temperature on the GenFast-like column with glass fiber GF/L-6^{™} media (Whatman), control vs. FTA®-like solution (FTA® treated) (MW = molecular weight standard).
**Figure 7** is an agarose gel electrophoresis analysis of the quality of the DNA isolated after three and one-half months storage at room temperature on the silica gel QIAamp Mini Kit column (Qiagen).
**Figure 8** is an agarose gel electrophoresis analysis of the quality of the DNA isolated after three months storage at room temperature on column-based design hydrophilic polyvinylidinefluoride (PVDF) membrane (Whatman), control vs. FTA® treated vs. modified FTA® treated (MW = molecular weight standard; lanes 1-2: controls; lane 3: no sample (blank); lanes 4-5: FTA®-treated samples; lanes 6-9: modified FTA®-treated samples).

### Detailed Description of the Invention

Broadly, the present invention provides compounds and methods for using the compounds, such as those of FTA® technology (Whatman, Inc.), for the preservation and storage of nucleic acid (NA) from blood or other biological samples on the media of any solid phase based nucleic acid purification system (including commercial kits and in-house devices). For example, in one embodiment this result is achieved by applying and drying FTA® chemicals on the solid phase media after the loading of the sample or after a given step of a nucleic acid extraction procedure.

The present invention also provides compounds and methods for enhancing an amount of stored NA in excess of quantities that are considered to be optimal for nucleic acid purification systems. It does not limit the FTA® technology to the times and temperature ranges of the given sample storage procedure.

The method is fast, requiring only a few buffers and a simple heat elution step to recover the DNA. The method does not rely on chaotrophic salt, ion exchange, or affinity extraction procedures.

In some instances, the solid phase media may physically trap the retentate. Alternatively, however, the retentate may interact chemically with the media. It is possible that a combination of adsorption and absorption may take place.

The present method provides a quick, simplified, cost effective method for storing, and subsequently isolating, nucleic acids using a wide range of commercially available solid phase media, which until now have been considered inappropriate for storage. The technique is not manually intensive or technique-dependent and does not utilize hazardous chemicals. The nucleic acid produced in accordance with the present invention is capable of multiple downstream processing.

In one aspect, the present invention provides a method for isolating and storing nucleic acid, comprising:
a. providing a solid phase medium;
b. applying a sample comprising cells containing nucleic acid to the solid phase medium;
c. retaining the cells with the solid phase medium as a cellular retentate and removing contaminants;
d. contacting the cellular retentate with a solution comprising a surfactant or detergent;
e. lysing the cellular retentate to form a cell lysate while retaining the cell lysate in the medium, the cell lysate comprising the nucleic acid;
f. drying the solid phase medium with the cell lysate comprising the nucleic acid; and
g. storing the dried solid phase medium with the nucleic acid,
h. eluting the nucleic acid from said medium, wherein the storage of the nucleic acid in step g has a duration of at least one week.

In one preferred embodiment, prior to drying step f, the solid phase medium with the nucleic acid is washed to remove contaminants while retaining the nucleic acid in the solid phase medium.

In another preferred embodiment, prior to eluting step h, the dried solid phase medium with the nucleic acid is washed to remove contaminants while retaining the nucleic acid in the solid phase medium.

In a preferred embodiment, the dried solid phase medium with the nucleic acid in step g is maintained substantially at a temperature of 5°C to 40°C.

The storage of the nucleic acid in step g has a duration of at least one week. Preferably, it has a duration of at least one month; more preferably, it has a duration of at least three months. Still more preferably, the storage of the nucleic acid in step g has a duration of at least five months.

In a preferred embodiment, the solid phase medium comprises a filter comprising a plurality of fibers. Preferably, the filter has a substantially disordered structure. Preferably, the fiber diameters are in the range of from 1 µm to 10 µm. Preferably, the filter comprises one or more pores having a pore size from about 0.2 µm to about 2.7 µm.

In a preferred embodiment, the solid phase medium comprises:
a. a glass or silica-based solid phase medium;
b. a plastics-based solid phase medium; or
c. a cellulose-based solid phase medium.

Preferably, the solid phase medium is selected from one of the following: glass, glass fiber, glass microfiber, silica, silica gel, silica oxide, cellulose, nitrocellulose, carboxymethylcellulose, polyester, polyamide, carbohydrate polymers, polypropylene, polytetrafluoroethylene, polyvinylidinefluoride, wool, or porous ceramics.

In one preferred embodiment, the surfactant or detergent of step d comprises an anionic surfactant or detergent, preferably, sodium dodecyl sulfate, still more preferably wherein the concentration of the sodium dodecyl sulfate is between about 0.5% and about 5% weight/volume (w/v).

In another preferred embodiment, the solution of step d further comprises:
ii. a weak base; and
iii. a chelating agent.

Preferably, in addition to the above, the solution of step d further comprises:
iv. uric acid or a urate salt.

In a preferred embodiment, the cellular retentate comprises condensed material from the nucleus.

In a particularly preferred embodiment, the cellular retentate comprises intact whole cells and step e comprises:
i. rupturing the intact whole cells retained by the solid phase medium to leave condensed material from the nucleus retained by the medium; and
ii. lysing the condensed material from the nucleus to form the cell lysate containing the nucleic acid.

In a preferred embodiment, the composition and dimensions of the solid phase medium are selected so that the nucleic acid is retained by the medium in step e substantially by non-ionic interactions, preferably dipole-dipole interactions, dipole-induced dipole interactions, dispersion forces, or hydrogen bonding.

Preferably, the retaining step e is further defined as physically retarding the movement of the nucleic acid through the solid phase medium.

Preferably, the solid phase medium is capable of retaining the cells and the nucleic acid in the absence of a chaotrope.

Preferably, step b further comprises concentrating the cells in the solid phase medium.

In one preferred embodiment, the nucleic acid is heated to an elevated temperature of 65°C to 125°C prior to eluting step h and more preferably to an elevated temperature of 80°C to 95°C prior to eluting step h.

Preferably, the cells are selected from the group consisting of white blood cells, epithelial cells, buccal cells, tissue culture cells, semen, vaginal cells, urinary tract cells, plant cells, bacterial cells, and colorectal cells.

In a particularly preferred embodiment, the cells are white blood cells and the method further comprises applying whole blood to the solid phase medium, optionally lysing the red blood cells therefrom, optionally washing the solid phase medium to remove contaminants, and obtaining the cell lysate from the white blood cells.

More preferably, the sample comprises blood cells and the dimensions of the solid phase medium are selected so that the majority of the cells retained in step c comprise white blood cells.

In a preferred embodiment, the nucleic acid comprises DNA or RNA, more preferably genomic DNA.

In another aspect, the present invention provides a method for isolating and storing nucleic acid, comprising:
a. providing a solid phase medium;
b. applying a sample comprising cells containing nucleic acid to the solid phase medium;
c. retaining the cells with the solid phase medium as a cellular retentate and removing contaminants;
d. contacting the cellular retentate with a solution comprising:
   i. a weak base;
   ii. a chelating agent; and
   iii. an anionic surfactant or detergent;
e. lysing the cellular retentate to form a cell lysate while retaining the cell lysate in the medium, the cell lysate comprising the nucleic acid;
f. drying the solid phase medium with the cell lysate comprising the nucleic acid;
g. storing the dried solid phase medium with the nucleic acid; and
h. eluting the nucleic acid from said medium, wherein the storage of the nucleic acid in step g has a duration of at least one week.

In a further aspect, the present invention provides a method for isolating and storing DNA, comprising:
a. providing a solid phase medium, wherein the solid phase medium comprises a filter comprising a plurality of fibers, wherein the fibers comprise:
   i. glass or silica-based fibers;
   ii. plastics-based fibers; or
   iii. nitrocellulose or cellulose-based fibers;
b. applying a sample comprising cells containing DNA to the solid phase medium;
c. retaining the cells with the solid phase medium as a cellular retentate and removing contaminants;
d. contacting the cellular retentate with a solution comprising:
   i. a weak base;
   ii. a chelating agent; and
   iii. an anionic surfactant or detergent;
e. lysing the cellular retentate to form a cell lysate while retaining the cell lysate in the medium, the cell lysate containing DNA;
f. drying the solid phase medium with the cell lysate comprising the DNA;
g. storing the dried solid phase medium with the DNA at a temperature of 5°C to 40°C;
h. heating the DNA with the solid phase medium to an elevated temperature of 65°C to 125°C; and
i. eluting the nucleic acid from said medium, wherein the storage of the nucleic acid in step g has a duration of at least one week.

It is preferred that the retentate be lysed while entrapped within the solid phase media. However, it should be understood that the method according to the present invention encompasses also an embodiment where substantially all or some of retentate is lysed while retained by, but not entrapped within, the solid phase media, including on the surface of the media.

In one aspect of the present invention, the cell retentate comprises intact whole cells as well as, or instead of, cell debris. Advantageously, the intact whole cells may be treated, while being retained by the solid phase media, by the application of a detergent to the media. Any detergent may be used, provided that it has the effect of rupturing or "peeling away" the cell membrane. Condensed nuclear material or nucleic acid is retained by the media. Preferably the detergent is selected from sodium dodecyl sulfate (particularly 0.5% - 5% weight-by-volume SDS), sodium lauryl sarcosinate (particularly 0.5% - 5% w/v SLS), or other commercially available detergents, such as TWEEN^{™} 20 (particularly 0.5% - 5% volume-by-volume TWEEN^{™} 20), lauryl dodecyl sulfate (particularly 0.5% - 5% w/v LDS) or TRITON^{™} e.g., TRITON^{™} X-100 (particularly 0.5% - 5% v/v TRITON™). Alternatively, 3-[(3-cholamidopropyl)dimethylammonio]-1-propane-sulfonate ("CHAPS"), may be used. The amount of detergent employed is sufficient to lyse cell membranes, but not so much as to denature DNA. Suitable amounts are generally 0.1% to 10% by weight (w/v) or by volume (v/v) and preferably 0.2% to 7% w/v or v/v and more preferably 0.5% to 5% w/v or v/v of SDS, TWEEN^{™} or TRITON^{™}. Most preferably the detergent is 0.5% to 5% w/v SDS.

While the addition of detergent to the retentate is preferable, the present method may be carried out without the addition of a detergent by using other known lysing agents, such as low-salt non-isotonic solutions, such as ethanol or sucrose. However, applying a detergent to the retentate while the retentate is retained by the solid phase medium increases the yield and purity of the DNA product.

In addition to rupturing the intact whole cells, the detergent also has the function of washing out protein and heme (haem) which may have been retained by the solid phase medium.

With regard to lysis of the cell nuclei, in one aspect of the invention, the nuclei are lysed, exploded, or ruptured to form a lysate containing nucleic acid by the addition of a low-salt, non-isotonic buffer, such as a hypotonic buffer. Preferably, the low salt buffer is
Per 1 liter -
- Measure 500 ml purified water
- Add 10.0 ml (9.9-1.1 ml) 1M Tris (Whatman WB420003)
- Add 0.596 g (0.595-0.597 g) KCl (Whatman WB410015)
- Add 0.29 g (0.28-0.30 g) MgCl₂ (Whatman WB410014)
- Mix to dissolve
- Add 10.0 ml NFB (alternatively, fetal calf serum may be used) and mix
- Add 0.5 g (0.49-0.51 g) Na-Metabisulfite (Whatman WB410055) and mix
- Add water to 1000 ml and mix well.
- Aseptically filter solution through an 0.2 µm filter in a class II safety cabinet
- Store at room temperature;
10 mM Tris-HCl, 1 mM EDTA, pH 7.6-8 ("AE,"); 10 mM Tris-HCl, 0.1 mM EDTA, pH 8 ("TE⁻¹"); or water. Other suitable lysis solutions include any detergent-containing solutions in which the detergent may be cationic, anionic or neutral. Chaotrope-containing solutions, preferably buffers may also be used. The lysis solution lyses or bursts open the condensed nuclear material to release the nucleic acid. It will be understood by the skilled person, however, that lysing the nuclei to form a lysate containing nucleic acid also can be achieved by other methods, for example, by heating.

Alternatively, after lysis, the retentate comprises freed nucleic acids.

Lysis, whether of cells or of nuclei, and the removal of the contaminating materials resulting from lysis may take place sequentially, such as lysis of cells followed by removal of non-nucleic materials or contents or lysis of nuclei followed by removal of non-nucleic acids. Alternatively, the lysis and removal steps may occur simultaneously or may overlap.

The chemical composition of the solution, which preferably comprises a surfactant or detergent and more preferably comprises a weak base, a chelating agent, and an anionic surfactant or detergent, facilitates the lysis of whole cells and the subsequent capture of the released nucleic acids. The chemical composition further aids in their long term storage. The composition of the solution is such that the rapid purification of the captured nucleic acid can be carried out. That is, the solution itself allows for the release of nucleic acid by an elution step thereby providing a soluble nucleic acid fraction. As discussed in more detail below and exemplified in the following examples, the present invention is most efficient with regard to elution of total DNA from the sample. However, nucleic acid and nucleic acid populations can be specifically eluted.

In embodiments comprising a filter membrane, it is preferred that the solution comprises a chemical composition that is able to sorb to the aforementioned filter membrane. The composition of the solution is preferably as described and relates to that outlined in US Patents 5,756,126, 5,807,527, and 5,496,562.

In one embodiment, the preferred solution includes a protein denaturing agent and a free radical trap. The denaturing reagent can be a surfactant that will denature proteins and the majority of any pathogenic organisms in the sample. Anionic detergents are examples of such denaturing reagents, and may be used alone. The chemical solution can include a weak base, a chelating agent, and the anionic surfactant or detergent, and optionally uric acid and urate salt as discussed in detail in the above-cited United States Patent 5,807,527. More preferably, the weak base can be a Tris, trishydroxymethyl methane, either as a free base or as the carbonate, and the chelating agent can be EDTA, and the anionic detergent can be sodium dodecyl sulfate. Other solutions having similar function can also be utilized in accordance with the present invention.

In one preferred embodiment, the solution used in this aspect of this invention comprises the following:
(i) a monovalent weak base (such as "Tris", tris-hydroxymethyl methane, either as the free base or as the carbonate);
(ii) a chelating agent (such as EDTA, ethylene diamine tetracetic acid); and
(iii) an anionic detergent (such as SDS, sodium dodecyl sulfate); and optionally
(iv) uric acid or a urate salt.

An example of one preferred embodiment of the solution is an FTA® solution (Whatman, Inc.) comprising Tris, EDTA, SDS, and uric acid.

Although not vital for the short-term storage of DNA on the solid medium, the use of uric acid or a urate salt in accordance with this invention has been found to be particularly important for the long-term storage of DNA as this component performs a number of functions, including serving as a "free-radical" trap and a weak acid. As a free-radical trap, it preferentially accepts free radicals that would otherwise daage the base guanine in the DNA.

As previously described, the composition may include a base, optionally a monovalent weak base, to cause an alkaline pH between 8.0 and 9.5 to be imposed upon the blood that is placed upon the matrix. This is to ensure the proper action of the chelating agent in binding divalent metals. It is also to prevent the action of acid nucleases that are not so dependent on divalent metals. The base may be a weak organic base, such as Tris. Alternatively, an inorganic base such as an alkali metal carbonate or bicarbonate, for example sodium, lithium or potassium carbonate or bicarbonate, may be used.

The chelating agent is preferably a strong chelating agent such as EDTA, however a wide range of suitable strong chelating agents are commercially available. The function of the chelating agent is to bind the divalent metal ions, magnesium and calcium, and also to bind transition metal ions, particularly iron. Both calcium and magnesium are known to promote DNA degradation by acting as co-factors for enzymes. The metal ions such as iron, that readily undergo oxidation and reduction also damage nucleic acids by the production of free radicals.

The anionic surfactant or detergent is included in the composition of this aspect of the invention as the primary denaturing agent. The anionic detergent may be used alone or in combination with the weak base, the chelating agent, and optionally, uric acid or a urate salt.

Any strong anionic detergent that binds to and denatures proteins is suitable, and as well as SDS mentioned above, other detergents such as sodium lauryl sarcosinate may also be used. This anionic detergent causes most pathogens to be inactivated due to the non-specific destruction of the secondary structure of their coat proteins, their internal proteins, and any membranes they may be dependent upon. There are exceptions, since the anionic detergent does not inactivate the most resistant bacterial spores, nor does it inactivate some extremely stable enteric virions. However, these exceptions are agents that are already likely to be transferred by ordinary contact and there is currently no great concern that these agents constitute a risk from blood.

The anionic detergent can be selected from the group including sodium dodecyl sulfate (SDS). SDS can be obtained in various forms, such as the C₁₂ form and the lauryl sulfate. Other anionic detergents can be used, such as alky aryl sulphonates, sodium tetradecylsulphate long chain (fatty) alcohol sulphates, sodium 2-ethylhexysulphate olefine sulphates, sulphosuccinates or phosphate esters. The anionic detergent, such as SDS, can be applied to the filter matrix at varying concentrations.

Generally, 0.1%-10% SDS can be used in accordance with the present invention. For example, increased concentrations of SDS, up to 10%, which cannot be accommodated within an FTA® cocktail, as set forth in the patents discussed above, provided greater critical micelle concentration, which generates greater lysing capability and thus greater yield of target nucleic acid, as demonstrated in the example section set forth below. A preferred SDS concentration is achieved in the 0.5% - 5% SDS concentration range for glass microfiber in order to enrich for and purify different plasmid populations directly from liquid cultures, such as in a packed column, a filter column or a multi-well format, such formats being well known in the art.

Suitable materials for the solid phase media include glass fiber or any silica-based or derived solid phase media and plastic-based solid phase media, for example, polyester and polypropylene based media.

Certain materials, including glass microfiber, polyethylene, polyester, or polypropylene, make it possible to isolate nucleic acid in the absence of a chaotrope. It is preferred that the composition and dimensions are selected so that the solid phase medium is capable of retaining the cells and the nucleic acid substantially in the absence of a chaotrope. It has been surprisingly found by the applicant that with certain filter materials, including glass microfiber, polyethylene, polyester, or woven or polypropylene, it is possible to isolate nucleic acid in the absence of a chaotrope. This goes against the conventional wisdom of those skilled in the art of the invention.

Preferably, the solid phase medium is of a depth that is sufficiently large to entrap the cells and the nucleic acid within the medium without substantial loss. The present method is scalable so that any surface area of the filter and thus any filter diameter may be used.

The solid phase medium of the present invention can be capable of releasing the generic material immobilized thereto by a heat elution. In one preferred embodiment, such a heat elution is accomplished by the exposure of the medium having the genetic material stored thereon to heated eluting solution or water, the eluting solution or water being nuclease free. In preferred embodiments, the solid phase medium is porous and presents a vast surface area upon which the nucleic acid is bound.

The solid phase medium of the invention is such that at any point during a storage regime, it allows for the rapid purification of immobilized nucleic acid. The immobilized nucleic acid is collected in the form of a soluble fraction following a simplified elution process, during which immobilized nucleic acid is released from the solid phase medium of the invention. The solid phase medium of the invention yields nucleic acid of sufficient quality that it does not impair downstream analyses such as polymerase chain reaction (PCR), ligase chain reaction (LCR), transcription mediated amplification (TMA), reverse transcriptase initiated PCR, DNA or RNA hybridization techniques, sequencing, and the like.

In one embodiment, the solid phase medium comprises a filter or filter membrane. If the filter is fibrous, the solution above preferably coats the filter fibers, rather than simply coating the filter surface. Alternatively, the solution can impregnate the fibers.

The term "filter," "filter membrane" or "matrix" as used herein means a porous material or filter media formed, either fully or partly from glass, silica, silica gel, silica oxide, or quartz, including their fibers or derivatives thereof, but is not limited to such materials. Other materials from which the filter membrane can be composed also include, but are not limited to, cellulose-based (e.g., cellulose, nitrocellulose, carboxymethylcellulose), hydrophilic polymers including synthetic hydrophilic polymers (e.g., polyester, polyamide, carbohydrate polymers), polytetrafluoroethylene, porous ceramics, Sephacryl S-500, wool, and diatomaceous earth (silica oxide). Alternatively, one or more filters may be crushed, broken, homogenized or the like, and used to pack a tube, column or multiwell plate. In this instance, the filter material may be used to form a sludge or suspension in, e.g., a buffer or other solution.

Preferred examples of solid phase media to be used in the present invention include, but are not limited to, GF/L-6^{™} glass fiber media (Whatman, Inc.), DBS-1000^{™} glass fiber media (Whatman, Inc.), GenFast columns (Whatman, Inc.), PVDF media (Whatman, Inc.), QiaAmp® columns (Qiagen GmbH), Wizard® columns (Promega), and Generation® columns (Gentra). Further examples include, but are not limited to, nitrocellulose membranes (Whatman, Inc.; Toronto; Schleicher & Schuell), cellulose nitrate membranes (Arbor Tech), cellulose acetate membranes (Toronto), and nylon membranes (Whatman, Inc.; Standard and SP; Osmonics). Additional examples are known in the art, for example, WO 00/21973, U.S. Patent 5,234,809, European Patent No. 0,389,063,

Additional preferred embodiments include, but are not limited to, plasmid prep kits, whether in a column format or in a high-throughput 96-well format (vacuum or centrifuge), comprising a wide range of materials. Examples of commercially available high-throughput plasmid prep kits include, but are not limited to, kits including DNA-binding matrices (DNAce 96 Plasmid kit (Bioline); Perfectpre-96 Spin (Eppendorf Scientific, Inc.)), silica membranes (Aurum Plasmid 96 kit (Bio-Rad Laboratories); Nucleospin Multi-96 (Clontech Laboratories, Inc.); Perfectprep 96 Vac DB (Eppendorf Scientific, Inc.) Wizard SV 96 Plasmid DNA Purification System (Promega Corp.)), silica gel membrane (QIAprep 96 Turbo Miniprep kit (Qiagen)); silica matrix (96-well prep Express (Qbiogene); RPM Turbo 96 kit (Qbiogene)); and anion exchange membrane (QIAwell 96 Ultra Plasmid kit (Qiagen)).

An automated plasmid prep device may be used to facilitate rapid isolation of nucleic acids prior to storage or to facilitate elution following storage, in addition to reducing the possibility of sample contamination. Examples of automated plasmid prep devices include, but are not limited to, AutoGenprep 960 (AutoGen, Inc.), PERFECTprep Process Platform (Brinkmann), RevPrep (GeneMachines), Miniprep Workstation (Hudson), AutoPrep-12 (ThennoHybaid), Mini-prep 24 (MacConnell Research), RoboPrep 2500/3500/4800 (MWG Biotech, Inc.).

Similarly, high-throughput automated isolation or elution techniques and equipment could be used for isolating genomic DNA and RNA.

Preferably, the media used for the filter membrane of the invention includes any material that does not inhibit the above-described solution and which does not inhibit the storage, elution and subsequent analysis of nucleic acid-containing material added to it. This includes flat dry matrices or a matrix combined with a binder. Examples of binders include, but are not limited to, polyethylene glycol (PEG), polyvinyl alcohol (PVA), polyvinyl pyrrolidine (PVP), and other long-chain alcohols.

It is preferred that the filter membrane of the invention be of a porous nature to facilitate immobilization of nucleic acid. In a preferred embodiment, the filter comprises a plurality of fibers and has a substantially disordered structure. Preferably, the fiber diameters are in the range of about 1 µm to about 10 µm. Preferably, the fiber matrix comprises one or more pores having a pore size from about 0.2 µm to about 2.7 µm.

In a preferred embodiment, the filter composition and dimensions are selected so that the nucleic acid is retained by the filter substantially in the absence of ionic interaction, more preferably by physical retardation of the movement of the nucleic acid through the filter.

In a preferred embodiment, the filter composition and dimensions are selected so that the nucleic acid is retained by the filter in the form of a web.

"Nucleic materials" and "materials from the nucleus" include the nuclear envelope and the contents of the nucleus, including genomic DNA ("gDNA") or plasmid DNA. The "non-nucleic acid contents of the nucleus" include the components of the nuclear envelope and any other proteins or other substances of the nucleus that are not nucleic acids.

"Nucleic acids" include deoxyribonucleic acids (DNA) and ribonucleic acids (RNA) of various types. "Genetic material" comprises genomic DNA ("gDNA"), which is one type of DNA and encodes genetic information.

It is preferred that the nucleic acid comprises a polynucleotide.

While the method is applicable to any nucleic acid, it is preferred that that the nucleic acid comprises DNA, especially genomic DNA. While it is indicated in this preferred method that genomic DNA is the desired target compound, it is possible to use the method of the present invention to isolate RNA from an RNA-containing sample.

Aside from the elution steps described below, the temperature for the non-elution steps is usually ambient temperature, typically in the range from 5°C to 40°C, preferably in the range from 10°C to 40°C, more preferably in the range of 15°C to 30°C.

In a preferred embodiment, the storage step is at ambient temperature ("room temperature") and humidity. Typically, the temperature is in the range from 5°C to 40°C, preferably in the range from 10°C to 40°C, more preferably in the range of 15°C to 30°C.

Storage of the nucleic acid with the dried solid phase media may be maintained for weeks, or months. In a preferred embodiment, the nucleic acid is capable of being stored for at least one month, even more preferably for three months, and still more preferably for five months.

It is preferred also that the composition and dimensions of the solid phase media are selected so that the nucleic acid is capable of being eluted at a pH of from pH 5 to 11 or preferably from pH 5.8 to 10. This is advantageous in the present method because elution of the product nucleic acid in a more highly alkaline medium potentially can degrade the product. Accordingly, one preferred pH for elution is from 7 to 9.

Any solution at any pH which is suitable for eluting the nucleic acid from the present filter may work. Preferred elution solutions include sodium hydroxide (NaOH ) (1 mM to 1 M), sodium acetate (Na acetate) (1mM to 1M), 10mM 2-[N-morpholino]-ethanesulfonic acid (MES) (pH 5.6), 10mM 3-[cyclohexylamino]-1-propanesulfonic acid (CAPS) (pH 10.4), TE (10mM Tris HCL (pH8) + 1mM EDTA), TE⁻¹ (10 mM Tris; 0.1 mM EDTA; pH 8), sodium dodecyl sulfate (SDS) (particularly 0.5% SDS), TWEEN^{™} 20 (particularly 1% TWEEN^{™} 20), LDS (particularly 1% lauryl dodecyl sulfate (LDS)) or TRITON^{™} (particularly 1% TRITON™), water and 10mM Tris. In typical applications, these solutions yield approximately the same quantity of nucleic acid. Total yields of nucleic acid are higher when eluted in a high volume of elution solution.

Eluting the nucleic acid, in other words releasing the nucleic acid from the solid phase media, may be affected in several ways. The efficiency of elution may be improved by putting energy into the system during an incubation step to release the nucleic acid prior to elution. This may be in the form of physical energy (for example by agitating) or heat energy. The incubation or release time may be shortened by increasing the quantity of energy put into the system.

It is possible to elute nucleic acid from the solid phase media at room temperature, but the efficiency of elution may be improved by putting energy into the system during an incubation step to release the nucleic acid prior to elution. This may be in the form of heat energy. Preferably, heat energy is put into the system by heating the nucleic acid to an elevated temperature for a predetermined time, while it is retained by the solid phase media, prior to elution, but not at a sufficiently high temperature or for such a time as to be damaged. More preferably, the nucleic acid is heated to an elevated temperature in the range of 40°C to 125°C, even more preferably in the range of from 80°C to 95°C. Most preferably, the nucleic acid is heated to an elevated temperature of about 90°C, advantageously for about 10 minutes.

Alternatively, elution buffer that has already been heated to an elevated temperature may be used in lieu of, or more preferably, in addition to, elution at an elevated temperature.

In a preferred embodiment, the elution step described above takes place as follows:
(a) heating an elution buffer to an elevated temperature of 40°C to 125°C;
(b) adding the elution buffer to the solid phase media;
(c) heating the solid phase media and elution buffer to an elevated temperature of 40°C to 125°C;
(d) eluting the nucleic acid; and
(e) repeating steps (a)-(d) together at least once.

More preferably, the elevated temperature is in the range from 80°C to 95°C. Most preferably, the nucleic acid is heated to an elevated temperature of about 90°C, advantageously for about 10 minutes.

It should be noted that predominantly single stranded material will be produced from the present system. However, the ratio of double to single stranded DNA is dependent upon, and can be controlled by, the experimental conditions. Modifying the incubation regime using the parameters of time and temperature will alter this ratio, where a lower elution temperature over a longer time period will produce a high proportion of double stranded DNA. A higher elution temperature over a shorter period of time also will also produce a higher proportion of double stranded DNA.

Once the nucleic acid has been heated to an elevated temperature while retained by the filter, it is not necessary to maintain the nucleic acid at the elevated temperature during elution. Elution itself may be at any temperature. For ease of processing, it is preferred that, where the nucleic acid is heated to an elevated temperature while retained by the filter, elution will be at a temperature lower than the elevated temperature. This is because when heating has been stopped, the temperature of the nucleic acid will fall over time and also will fall as a result of the application of any ambient temperature eluting solution to the filter.

It is preferred that the method be conducted substantially in the absence of a chaotrope.

In many instances, blood samples are applied to a column comprising a solid phase medium. In a preferred embodiment, blood samples are treated with a red blood cell lysis solution. Typical red blood cell lysis solutions that may be used in the method of the invention include those set out in Table 1. A preferred solution is 0.83% w/v ammonium chloride; 0.16% w/v ammonium carbonate; 0.1 mM EDTA. Red cell lysis is not absolutely necessary as the filter will allow intact red cells to pass through. However, inclusion of the red blood cell lysis solution leads to a cleaner final product.

Reagents suitable for analysis of liberated DNA can include any reagent suitable for PCR, processing of DNA, digesting or subcloning of DNA or portions thereof, sequencing of DNA, restriction fragment length polymorphism ("RFLP") analysis, Southern blotting and any other downstream applications generally found within the scope of DNA analysis. Reagents include, but are not limited to, probes, primers, restriction enzymes, buffers, proteins, indicators, and any other reagent useful for analysis of DNA.

Reagents suitable for analysis of liberated RNA, such as mRNA, can include any reagent suitable for reverse transcription, processing, Northern blotting, and any other downstream applications generally found within the scope of RNA analysis. Reagents include, but are not limited to, probes, primers, restriction enzymes, buffers, proteins, indicators, and any other reagent useful for analysis of RNA.

It has been found that the present method substantially improves the yield and purity of the nucleic acid product. Furthermore, the present method provides a quick, simplified, cost effective method for nucleic acid purification that is not manually intensive or technique-dependent and does not utilize hazardous chemicals. The nucleic acid produced in accordance with one embodiment of the present invention is capable of multiple downstream processing. Optionally, the nucleic acids retained by the filter may be washed with any suitable wash solution. Preferably, the nucleic acid retained by the filter is washed with a buffer having a pH in the range 5.8 to 10, more preferably in the range 7 to 8. In particular, washing with water or a low salt buffer such as TE⁻¹ (10 mM Tris HCL (pH8) with 100µm EDTA) is preferred. The washing step may occur prior to or at the same time as elution. Washing increases the yield and purity of the nucleic acid product. The washing step removes any remains of the cell material-lysis solution which may be problematic in downstream processing.

Alternatively, an appropriate washing buffer can be selected from the group including Tris/EDTA; 70% ethanol; STET (0.1 M NaCl; 10 mM Tris-Cl, pH 8.0; 1 mM EDTA, pH 8.0; 5% Triton X-100); SSC (20X SSC = 3 M NaCl; 0.3 M sodium citrate; pH 7.0 with NaOH); SSPE (20X SSPE = 3 M NaCl; 0.2 M NaH₂PO₄-H₂O; 0.02 M EDTA; pH 7.4); FTA^{™} purification reagent, and the like. In particular, washing with water or a low salt buffer such as TE⁻¹ (10 mM Tris HCL (pH8) with 100µm EDTA) is preferred.

A washing step, such as with various buffers set forth in the example section, but not limited thereto, may be done before or after cell lysis. When the washing step is performed after cell lysis, the solid phase medium then physically captures the nucleic acid within the intrastaces thereof. In addition, a washing step may be performed following storage just prior to elution.

In a preferred embodiment, the method includes a washing step prior to the cell lysis step in order to remove contaminants, such as debris, including cell debris. In another preferred embodiment, the method includes a washing step following the cell lysis step and prior to the step of drying the solid phase membrane, likewise to remove contaminants. In yet another preferred embodiment, the method includes a washing step following the storage step and prior to the elution step.

The source of the nucleic acid can be a biological sample containing whole cells. The whole cells can be, but are not restricted to, blood, bacterial culture, bacterial colonies, tissue culture cells, saliva, urine, drinking water, plasma, stool samples, semen, vaginal samples, sputum, and plant cell samples. The samples can be collected by various means known in the art, transported to the solid phase medium, and then applied thereto. Alternatively, the solid phase medium can be in the form of a sampling device, such as a swab, sheet material, ball, or the like and the sample can be obtained directly from the source. In other words, the solid phase medium can be in the form of a device which can swipe or otherwise obtain the cell sample from a source. The source can be a sample tube containing a liquid sample; an organ, such as a mouth, ear, or other part of a human or animal; a sample pool, such as a blood sample at a crime scene or the like; whole blood or leukocyte-reduced blood; or other various sources of cells known in the scientific, forensic, and other arts. The applying step can be achieved by applying whole cells to the solid phase medium. The nucleic acid may be eukaryotic or prokaryotic and may also include plasmid DNA.

In general, the present method may be applied advantageously to any whole cell suspension. Cells particularly amenable to the present method include bacterial cells, yeast cells, plant cells and mammalian and other animal cells, such as white blood cells, epithelial cells, buccal cells, tissue culture cells, semen, vaginal cells, urinary tract cells, and colorectal cells. DNA has been obtained successfully from swabs, saline and sucrose mouthwashes and buffy coat samples.

Where the cells comprise white blood cells, it is preferred that the method further comprises applying whole blood to the solid phase, optionally lysing the red blood cells therefrom, optionally washing the solid phase to remove contaminants and obtaining the cell lysate from the blood cells. The whole blood can be fresh or frozen. Blood containing Na/EDTA, K/EDTA, and citrated blood all give similar yields. A 100µl sample of whole blood gives a yield of approximately 2-5µg of nucleic acids, a 500µl sample gives a yield of approximately 15-40µg of nucleic acids and a 10ml sample gives a yield of approximately 200-400µg of nucleic acids.

The present invention can find utility in many areas of genomics. For example, the present invention provides the capability to elute bound genetic material for the rapid purification of the genetic material to be utilized for a wide variety of commercially available solid phase media, such as solid phase media in a column, tube, or multiwell plate.

**Table 1. Solutions.**

| Reference | Vol Blood | Vol Lysis Solution | Composition | Treatment |
|---|---|---|---|---|
| Millar et al (1988) N.A.R 16:1215 | | 3ml | 10mM Tris- HCL pH 8.2 400mM NaCl 2mM EDTA | Treat o/n Prot K |
| Nelson & Krawetz (1992) Anal Biochem 207: 197-201 | 1 Vol | 5 Vol | 17mM Tris-HCl pH 7.65 140mM NH₄Cl | 37°C for 5min |
| Ramirez-Solis et al (1992) Anal Biochem 201:331-335 | 1 ml | 3ml | 155mM NH₄Cl 10mM NaHC0₃ | 4°C for 10-15min |
| Douglas et al (1992) Anal Biochem 201:362-365 | 1ml | 1ml of 2x RBC lysis | 1x:- 11% sucrose 10mM MgCl₂ 10mM Tris-HCl pH 7.5 1% Triton X-100 | pellet and wash with 1x |
| Linblom and Holmlund (1988) Gene Anal Techn 5:97-101 | 5ml | 10ml | 1% Triton X-100 320mM sucrose 1mM Tris-HCl pH7.5 5mM MgCl₂ | pellet/urea and phenol |
| | 0.2-2ml | 20ml | 20mM Tris-HCl | Used with |
| | | | pH 8.0 | Leukosorb |
| | | | 5mM EDTA | type filler |
| Hermmann and Frischauf(1987) in Guide to | 10ml | 30ml | 155mM NH₄Cl 10mM NH₄C0₃ | ice 15min, spin |
| Molecular Cloning p180-183 | | | 0.1mM EDTA | |

### Examples

Human whole blood samples (400 - 1000 µl) were processed using original and modified versions of the Whatman Genomic DNA Purification System GenFast. 25- 50 µl FTA® solution (Whatman) were applied to each column after Nucleated Cell Capture and Red Cell Lysis Step. Then columns were left at room temperature for drying, storage and subsequent DNA extraction.

GenFast protocol was applied for recovering of the stored DNA, as described below.

### Study design for Examples 1-4:

- 0.5 - 1 ml of fresh or frozen human whole blood was applied onto homemade columns packed with different types of Leukoreduction (LR) glass fiber media;
- Hb and other undesirable blood components were washed out with red-cell lysing solution GenFast Solution 1 (Whatman, Inc.) [Solution 1 = 155.2 mM Ammonium Chloride, 10 mM Ammonium Carbonate, 0.10 mM EDTA];
- An FTA® solution (Whatman, Inc.) [(160 mM Tris; 7.8 mM EDTA, 2% w/v SDS, 0.672% w/v uric acid)] in a volume of 25-50µl was applied to the media with captured white blood cells on it (except as otherwise indicated);
- Columns were dried at room temperature or 37°C;
- Columns were stored at ambient conditions (room temperature, humidity) for 1, 5, or 20 days or for 3.5 months;
- At different points of storage DNA was isolated from the media using GenFast chemistry (GenFast Solutions 2, 3, 4) (Whatman, Inc.) [Solution 2 = 0.5% (w/v) of Sodium Lauryl Sulfate; Solution 3 = 8.0 (±0.1) mM Potassium Chloride, 3.0 (±0.1) mM Magnesium Chloride, 10 mM (±0.1 mM) Tris/HCl (pH 8.0), 1 % FCS (v/v), 0.05% (w/v) Sodium Metabisulfite; Solution 4 = 10 mM Tris/HCl; 1 mM EDTA];
- DNA quality and quantity was evaluated using UV/vis spectrometry, PicoGreen fluorescent dye, and agarose gel electrophoresis.

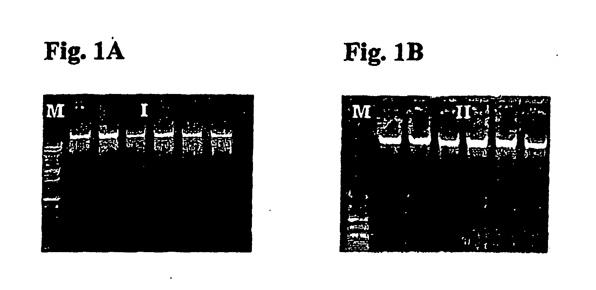

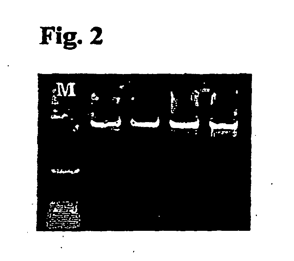

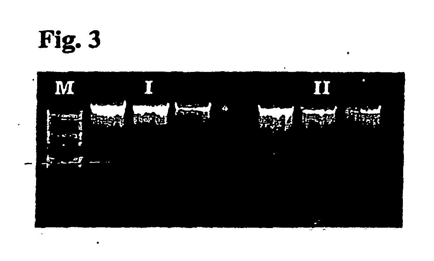

The following examples demonstrate the feasibility of extracting up to 78% of expected DNA yield (8 -12 µg DNA from 500 µl of blood) with up to 75% in double stranded form after 3.5 months of sample storage at room temperature. Stability of DNA in the storage process is presented the figures depicting photographs of the agarose gel electrophoresis after 1 (Fig. 1A), 5 (Fig. 1B), or 20 (Fig. 2) days, and 3.5 months (Fig. 3) of sample storage.

### Examples 1 and 2:

A sample of 0.5 ml frozen blood (#1) was applied onto glass fiber GF/L-6^{™} media in homemade columns. Storage time was 1 and 5 days. Results are shown in Table 2 and in Figures 1A-1B.

**Table 2. DNA Yield from the blood samples after 1 (I) and 5 (II) days of storage on the GF/L-6^{™} media at room temperature, detected by UV/vis spectroscopy.**

| I. DNA extraction after one day | | | | II. DNA extraction after five days storage of | | | |
|---|---|---|---|---|---|---|---|
| storage of the samples at room | | | | the samples at room temperature | | | |
| temperature | | | | | | | |
| Sample | ng | Extr | Yield, % to | Sample | ng DNA/µl | Extr | Yield, % to |
| | DNA | DNA, | Expected. | | | DNA, µg | Expected. |
| | /µl | µg | | | | | |
| 1 | 40 | 10 | 72 | 5 | 41 | 10 | 73 |
| 2 | 43 | 10 | 76 | 6 | 42 | 10 | 75 |
| 3 | 47 | 11 | 84 | 7 | 44 | 11 | 78 |
| Average | 43.1 | 10.3 | 77.2 | Average | 42.1 | 10.1 | 75.5 |
| SD | 2.74 | 0.66 | 4.91 | SD | 1.27 | 0.31 | 2.28 |

Figures 1A and 1B show the quality of extracted DNA from the blood samples after 1 (I) (Fig. 1A) and 5 (II) (Fig. 1B) days of storage on the GF/L-6^{™} media at room temperature, detected by 0.78% agarose gel electrophoresis (M = molecular weight standard).

### Example 3:

A sample of 0.5 ml fresh blood (#2) was applied onto glass fiber DBS-1000^{™} media in homemade column. Storage time was 20 days. Results are shown in Table 3 and in Figure 2.

**Table 3. DNA Yield from the blood samples after 20 days of storage on the DBS-1000^{™} media at room temperature, detected by UV/vis spectrophotometry and PicoGreen.**

| **Sample** | **dsDNA** | **Total DNA** | **dsDNA** | **Total DNA** | **dsDNA** | **Total DNA** |
|---|---|---|---|---|---|---|
| | µ**g/ml** | µ**g/ml** | µ**g/column** | µ**g/column** | **% of Expec.** | **% of Expec.** |
| **1** | 8.4 | 14.8 | 2.1 | 3.7 | 16 | 27 |
| **2** | 6.8 | 9.8 | 1.7 | 2.4 | 13 | 18 |
| **3** | 10.2 | 12.8 | 2.6 | 3.2 | 19 | 24 |
| **5** | 10.1 | 18 | 2.5 | 4.5 | 19 | 33 |
| **Mean** | 9 | 14 | 2 | 3 | 16 | 26 |
| **SD** | 1.41 | 3 | 0.35 | 0.75 | 2.61 | 5.55 |

Figure 2 shows the quality of extracted DNA from the blood samples after 20 days of storage on the DBS-1000^{™} media at room temperature, detected by 0.78% agarose gel electrophoresis (M = molecular weight standard).

### Example 4:

A sample of 1.0 ml fresh blood (#3) was applied onto DBS-1000^{™} media in homemade column. Storage time was 3.5 months. Results are shown in Table 4 and in Figure 3.

**Table 4. DNA Yield from the blood samples after 3.5 of storage on the DBS-1000^{™} media at room temperature, detected by UV/vis spectrophotometry and PicoGreen**

| **Sample** | Extract. Vol. | dsDNA | Total DNA | dsDNA | Total DNA | Total DNA | dsDNA |
|---|---|---|---|---|---|---|---|
| | ml | µg/ml | µg/ml | pglcolumn | µg/column | % of Exp | % of Eluted |
| **1** | **0.2906** | **34.6** | **46** | **10** | **13** | **38** | **77** |
| **2** | **0.2907** | **30** | **64** | **8.7** | **19** | **53** | **46** |

Figure 3 shows the quality of extracted DNA from the blood samples after 3.5 months of storage on the DBS-1000^{™} media at room temperature, detected by 0.78% agarose gel electrophoresis (M = molecular weight standard).

Based on the examples of the present invention, these results demonstrate the use of an embodiment of the present invention to archive NA at room temperature for following solid phase based purification technologies. Thus, the present invention enables use of solid phase based NA purification technologies to store, ship, and/or isolate the NA conveniently.

### Example 5: FTA®-composition for storage and isolation of the DNA from blood using 1-ml columns with different types of glass fiber media.

### Study design:

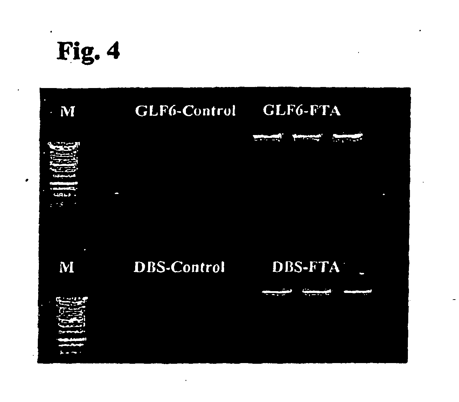

- 1 ml of blood was applied onto 1-ml columns with two different types of LR glass fiber media GF/L-6^{™} and DBS^{™} (Whatman, Inc.);
- Hb and other undesirable blood components were washed out with washing solution;
- FTA® solution was applied to one group of the columns (marked as GF/L-6-FTA® or DBS-FTA® below); no FTA® solution was applied to another group the columns (marked as GF/L-6^{™} -control or DBS^{™} -Control below).
- Columns were stored at ambient conditions (room temperature, humidity) for 1 month;
- DNA was isolated from the media using GenFast standard protocol;
- DNA quality and quantity was evaluated using UV/vis spectrometry, PicoGreen fluorescent dye (tables 5 and 6), and agarose gel electrophoresis (Figure 4).

### Results:

Figure 4 shows the quality of the DNA isolated after one month storage at room temperature on the GF/L-6^{™} and DBS^{™} media, control vs. FTA® treated, detected by 0.78% agarose gel electrophoresis (M = molecular weight standard).

The results presented in the Figure 4 and tables 5 and 6 demonstrate the excellent quality and quantity of the DNA isolated from the FTA® treated media, OD ratio was 1.8, about 80% of DNA was in double stranded (ds) form. DNA isolated from control, non-treated media was significantly deteriorated, OD ratio was 1.5 - 1.2, less then 20 % was in ds form. The amount of DNA isolated from control samples was 3 - 4 times less then that isolated from FTA®-treated columns.

**Table 5. DNA Yield from 1 ml blood after one month storage on the GF/L-6^{™} media (FTA® treated vs. control) at room temperature, detected by UV/vis spectroscopy.**

| **Sample** | **Total DNA** | **OD₂₆₀/OD₂₈₀** | **ds/ss** |
|---|---|---|---|
| | µ**g/column** | **Ratio** | **Ratio, %** |
| **GF/L-6^{™} -Control** | | | |
| Average | 6.2 | 1.55 | 22.7 |
| SD | 0.8 | 0.04 | 1.08 |

| **GF/L-6^{™}-FTA®** | | | |
|---|---|---|---|
| Average | 20 | 1.78 | 74 |
| SD | 1.9 | 0.03 | 2.1 |

**Table 6. DNA Yield from 1 ml blood after one month storage on the DBS^{™} media (FTA® treated vs control) at room temperature, detected by UV/VIS spectroscopy.**

| **Sample** | **Total DNA** | **OD₂₆₀/OD₂₈₀** | **ds/ss** |
|---|---|---|---|
| | µ**g/column** | **Ratio** | **Ratio, %** |
| **DBS^{™} -Control** | | | |
| Average | 4.4 | 1.34 | 20 |
| SD | 1.3 | 0.05 | 6 |

| **DBS^{™}-FTA®** | | | |
|---|---|---|---|
| Average | 16 | 1.79 | 80 |
| SD | 0.31 | 0.01 | 1.7 |

### Example 6: Quality of DNA samples extracted from a glass fiber column system after storage at room temperature for three and one-half months

### Study design:

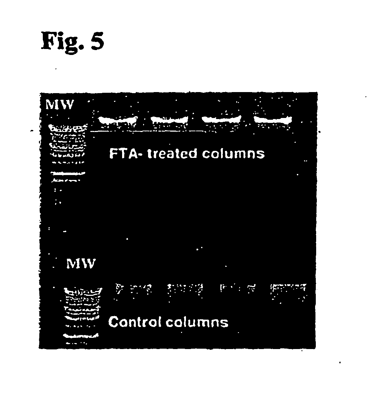

- 1 ml of whole, frozen blood was applied onto the filter media of 1-ml LR glass fiber GF/L-6^{™} spin columns;
- Hb and other undesirable blood components were washed once with washing solution (GenFast Solution 1).
- FTA® solution was applied to the media with the captured cells in the experimental columns, while no FTA® solution was applied to the control group;
- Columns were dried and stored at ambient conditions (room temperature, humidity) for 3.5 months;
- DNA was isolated from the media using GenFast standard protocol (Whatman, Inc.) as described above;
- DNA quality and quantity were evaluated using agarose gel electrophoresis (Figure 5) and PicoGreen fluorescent dye.

### Results:

Figure 5 shows the quality of the DNA isolated after 3.5 months storage at room temperature on the GF/L-6^{™} media, control (bottom) vs. FTA® treated (top), detected by 0.8% agarose gel electrophoresis (MW = molecular weight standard).

The results demonstrate the excellent quality (Figure 5) and quantity of the DNA isolated from the FTA® treated media, averaging 11 µg/column, with about 80% of the DNA in ds form (PicoGreen fluorescent dye). DNA isolated from control, non-treated media showed significant signs of deterioration (Figure 5).

### Example 7: Quality of DNA samples extracted from a glass fiber column system after storage at room temperature for five months

### Study design:

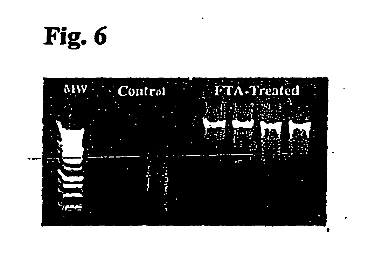

- 1 ml of whole, frozen blood was applied onto the filter media of 1-ml GenFast-like spin columns with glass fiber GF/L-6^{™} media and loaded by vacuum filtration;
- Hb and other undesirable blood components were washed once with washing solution (GenFast Solution 1).
- FTA®-like solution (without uric acid) (Whatman, Inc.) was applied to the media with the captured cells in the experimental columns (with FTA®-like solution comprising 2% SDS for two samples and FTA®-like solution comprising 4% SDS for two samples), while no FTA®-like solution was applied to the control group;
- Columns were dried and stored at ambient conditions (room temperature, humidity) for 5 months;
- DNA was isolated from the media using GenFast standard protocol (Whatman, Inc.), as described above;
- DNA quality and quantity were evaluated using agarose gel electrophoresis (Figure 6) and PicoGreen fluorescent dye.

### Results:

Figure 6 shows the quality of the DNA isolated after 5 months storage at room temperature on the GenFast-like spin columns with glass fiber GF/L-6^{™} media, control (Control) vs. FTA®-like solution-treated (FTA-Treated), detected by 0.78% agarose gel electrophoresis (MW = molecular weight standard). Of the four FTA-Treated lanes, the two left-hand FTA-Treated lanes (next to the Control lanes) were treated with FTA®-like solution comprising 4% SDS, while the two right-hand FTA-Treated lanes were treated with FTA®-like solution comprising 2% SDS. The amount of DNA in the FTA®-like solution-treated (FTA-Treated) lanes is approximately 100 ng DNA/band.

The results demonstrate the excellent quality (Figure 6) and quantity of the DNA isolated from the FTA® treated media, averaging 16 µg/column total DNA yield. DNA isolated from control, non-treated media showed significant signs of deterioration (Figure 6).

### Example 8: Quality of DNA samples extracted from a silica gel column system after storage at room temperature for three and one-half months

### Study design:

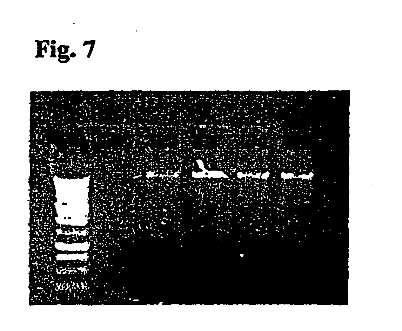

- 0.2 ml of whole, frozen blood was applied onto the filter media of 0.2-ml silica gel QIAamp Mini Kit (Qiagen) spin columns.
- Hb and other undesirable blood components were washed out with washing solution (GenFast Solution 1).
- FTA® solution was applied to the media with the captured cells in the experimental columns, while no FTA® solution was applied to the control group;
- Columns were dried and stored at ambient conditions (room temperature, humidity) for 3.5 months;
- DNA was isolated from the media using GenFast standard protocol (Whatman, Inc.), as described above;
- DNA quality and quantity were evaluated using agarose gel electrophoresis (Figure 7) and PicoGreen fluorescent dye.

### Results:

Figure 7 shows the quality of the DNA isolated after 3.5 months storage at room temperature on the FTA® treated QIAamp media detected by an 0.8% agarose gel electrophoresis (MW = molecular weight standard).

The results demonstrate the excellent quality (Figure 7) and quantity of the DNA isolated from the FTA® treated media, averaging 3.4 µg/column, with about 70% of the DNA in ds form (PicoGreen fluorescent dye).

### Example 9: Quality of DNA samples extracted from a PVDF column system after storage at room temperature for three and one-half months

### Study design:

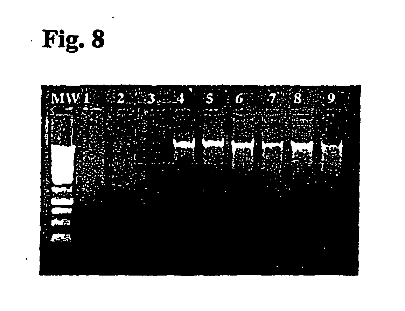

- 0.4 ml suspension of white blood cells (WBC; 10,000 cells/µl) was applied onto the filter media of 1.2 µm PVDF (polyvinylidinefluoride) hydrophilic membranes (Whatman) in a GenFast column-based design (Whatman, Inc.) by vacuum filtration;
- Columns were washed once with washing solution (GenFast Solution 1).
- FTA® solution (with uric acid) or modified FTA® solution (without uric acid) was applied to the media with the captured cells in the experimental columns, while no FTA® solution was applied to the control group;
- Columns were dried and stored at ambient conditions (room temperature, humidity) for 3.5 months;
- DNA was isolated from the media using GenFast standard protocol (Whatman, Inc.), as described above;
- DNA samples were diluted 10 times, and the quality and quantity of the DNA were evaluated using agarose gel electrophoresis (Figure 8) and PicoGreen fluorescent dye.

### Results:

Figure 8 shows the quality of the DNA isolated after 3 months storage at room temperature on the PVDF media, detected by 0.78% agarose gel electrophoresis (MW = molecular weight standard; lanes 1-2: Controls; lane 3: no sample (blank); lanes 4-5: FTA®-treated samples; lanes 6-9: modified FTA®-treated samples).

The results demonstrate the excellent quality (Figure 8) and quantity of the DNA isolated from the FTA® treated media and modified FTA® treated media, averaging 15 µg/column (about 60% of expected amount), with about 75% of the DNA in ds form (PicoGreen fluorescent dye). DNA isolated from control, non-treated media showed significant signs of deterioration (Figure 8).

The invention has been described in an illustrative manner, and it is to be understood that the terminology which has been used is intended to be in the nature of words or description, rather than of limitation.

Obviously, many modifications and variations of the present invention are possible in light of the above teachings.

### References

1. U.S. Patent No. 5,496,562, Burgoyne, Solid medium and method for DNA storage, 1996.
2. U.S. Patent No. 5,756,126, Burgoyne, Solid medium and method for DNA storage, 1998.
3. U.S. Patent No. 5,807,527, Burgoyne, Solid medium and method for DNA storage, 1998.
4. WO 00/21973 (2000), Mitchell et al., Isolation Methods and Apparatus (PCT/GB99/03337 (1999)).
5. U.S. Patent No. 5,658,548, Padhye et al., Nucleic Acid purification on silica gel and glass mixtures, 1997.

## Claims

1. A method for isolating and storing nucleic acid, comprising:
a. providing a solid phase medium;
b. applying a sample comprising cells containing nucleic acid to the solid phase medium;
c. retaining the cells with the solid phase medium as a cellular retentate and removing contaminants;
d. contacting the cellular retentate with a solution comprising a surfactant or detergent;
e. lysing the cellular retentate to form a cell lysate while retaining the cell lysate in the solid phase medium, the cell lysate comprising the nucleic acid;
f. drying the solid phase medium with the cell lysate comprising the nucleic acid;
g. storing the dried solid phase medium with the nucleic acid; and
h. eluting the nucleic acid from the solid medium;
wherein the storage of the nucleic acid in step g has a duration of at least one week.

2. The method of claim 1, wherein, prior to drying step f, the solid phase medium with the nucleic acid is washed to remove contaminants while the nucleic acid is retained in the solid phase medium.

3. The method of claim 1, wherein the dried solid phase medium with the nucleic acid in step g is maintained substantially at a temperature of 5°C to 40°C.

4. The method of claim 1, wherein, prior to eluting step h, the dried solid phase medium with the nucleic acid is washed to remove contaminants while the nucleic acid is retained in the solid phase medium.

5. The method of claim 1, wherein the storage of the nucleic acid in step g has a duration of at least one month.

6. The method of claim 1, wherein the storage of the nucleic acid in step g has a duration of at least three months.

7. The method of claim 1, wherein the storage of the nucleic acid in step g has a duration of at least five months.

8. The method of claim 1, wherein the solid phase medium comprises a filter comprising a plurality of fibers.

9. The method of claim 8, wherein the filter has a substantially disordered structure.

10. The method of claim 8, wherein the fiber diameters are in the range of from 1 µm to 10 µm.

11. The method of claim 8, wherein the filter comprises one or more pores having a pore size from about 0.2 µm to about 2.7 µm.

12. The method of claim 1, wherein the solid phase medium comprises:
a. a glass or silica-based solid phase medium;
b. a plastics-based solid phase medium; or
c. a cellulose-based solid phase medium.

13. The method of claim 1, wherein the solid phase medium is selected from one of the following: glass, glass fiber, glass microfiber, silica, silica gel, silica oxide, cellulose, nitrocellulose, carboxymethylcellulose, polyester, polyamide, carbohydrate polymers, polypropylene, polytetraflurorethylene, polyvinylidinefluoride, wool, or porous ceramics.

14. The method of claim 1, wherein the surfactant or detergent of step d comprises an anionic surfactant or detergent.

15. The method of claim 14, wherein the anionic surfactant or detergent comprises sodium dodecyl sulfate.

16. The method of claim 15, wherein the concentration of the sodium dodecyl sulfate is between about 0.5% and about 5% weight/volume.

17. The method of claim 14, wherein the solution of step d further comprises:
ii. a weak base, and
iii. a chelating agent.

18. The method of claim 17, wherein the solution of step d further comprises:
iv. uric acid or a urate salt.

19. The method of claim 1, wherein the cellular retentate comprises condensed material from the nucleus.

20. The method of claim 1, wherein the cellular retentate comprises intact whole cells and wherein step e comprises:
i. rupturing the intact whole cells retained by the solid phase medium to leave condensed material from the nucleus retained by the medium; and
ii.lysing the condensed material from the nucleus to form the cell lysate containing the nucleic acid.

21. The method of claim 1, wherein the composition and dimensions of the solid phase medium are selected so that the nucleic acid is retained by the medium in step e substantially by non-ionic interactions.

22. The method of claim 21, wherein the non-ionic interactions comprise dipole-dipole interactions, dipole-induced dipole interactions, dispersion forces, or hydrogen bonding.

23. The method of claim 1, wherein the retaining step e is further defined as physically retarding the movement of the nucleic acid through the solid phase medium.

24. The method of claim 1, wherein the solid phase medium is capable of retaining the cells and the nucleic acid in the absence of a chaotrope.

25. The method of claim 1, wherein step b further comprises concentrating the cells in the solid phase medium.

26. The method of claim 1, wherein the nucleic acid is heated to an elevated temperature of 65°C to 125°C prior to eluting step h.

27. The method of claim 1, wherein the nucleic acid is heated to an elevated temperature of 80°C to 95°C prior to eluting step h.

28. The method of claim 1, wherein the cells are selected from the group consisting of white blood cells, epithelial cells, buccal cells, tissue culture cells, semen, vaginal cells, urinary tract cells, plant cells, bacterial cells, and colorectal cells.

29. The method of claim 1, wherein the cells are white blood cells and the method further comprises applying whole blood to the solid phase medium, and obtaining the cell lysate from the white blood cells.

30. The method of claim 29, which further comprises lysing the red blood cells from the whole blood.

31. The method of claim 29 or claim 30, which further comprises washing the solid phase medium to remove contaminations.

32. The method of claim 1, wherein the sample comprises blood cells and the dimensions of the solid phase medium are selected so that the majority of the cells retained in step c comprise white blood cells.

33. The method of claim 1, wherein the nucleic acid comprises DNA or RNA.

34. The method of claim 1, wherein the nucleic acid comprises genomic DNA.

35. A method for isolating and storing nucleic acid, comprising:
a. providing a solid phase medium;
b. applying a sample comprising cells containing nucleic acid to the solid phase medium and concentrating the cells in the solid phase medium;
c. retaining the concentrated cells with the solid phase medium as a concentrated cellular retentate and removing contaminants;
d. contacting the concentrated cellular retentate with a solution comprising:
i. a weak base;
ii. a chelating agent; and
iii. an anionic surfactant or detergent;
e. lysing the concentrated cellular retentate to form a cell lysate while retaining the cell lysate in the solid phase medium, the cell lysate comprising the nucleic acid;
f. drying the solid phase medium with the cell lysate comprising the nucleic acid;
g. storing the dried solid phase medium with the nucleic acid for at least one week; and
h. eluting the nucleic acid from the solid phase medium.

36. A method for isolating and storing DNA; comprising:
a. providing a solid phase medium, wherein the solid phase medium comprises a filter comprising a plurality of fibers, wherein the fibers comprise:
i. glass or silica-based fibers;
ii. plastics-based fibers; or
iii. nitrocellulose or cellulose-based fibers;
b. applying a sample comprising cells containing DNA to the solid phase medium and concentrating the cells in the solid phase medium;
c. retaining the concentrated cells with the solid phase medium as a concentrated cellular retentate and removing contaminants;
d. contacting the concentrated cellular retentate with a solution comprising:
i. a weak base;
ii. a chelating agent; and
iii. an anionic surfactant or detergent;
e. lysing the concentrated cellular retentate to form a cell lysate while retaining the cell lysate in the solid phase medium, the cell lysate containing DNA;
f. drying the solid phase medium with the cell lysate comprising the DNA;
g. storing the dried solid phase medium with the DNA at a temperature of 5°C to 40°C for at least one week;
h. heating the DNA with the solid phase medium to an elevated temperature of 65°C to 125°C; and
i. eluting the DNA from the solid phase medium.

## Patentansprüche

1. Verfahren zur Isolierung und Lagerung von Nukleinsäure, umfassend:
a. Bereitstellen eines Festphasenmediums;
b. Aufbringen einer Probe, die Nukleinsäure enthaltende Zellen umfasst, auf das Festphasenmedium;
c. Zurückhalten der Zellen mit dem Festphasenmedium als zelluläres Retentat, und Entfernen von Kontaminanten;
d. Inkontaktbringen des zellulären Retentats mit einer Lösung, die ein Tensid oder Detergens umfasst;
e. Lysieren des zellulären Retentats, um ein Zelllysat zu bilden, bei gleichzeitigem Zurückhalten des Zelllysats im Festphasenmedium, wobei das Zelllysat die Nukleinsäure umfasst;
f. Trocknen des Festphasenmediums mit dem die Nukleinsäure umfassenden Zelllysat;
g. Lagern des getrockneten Festphasenmediums mit der Nukleinsäure; und
h. Eluieren der Nukleinsäure aus dem festen Medium;
wobei die Lagerung der Nukleinsäure in Schritt g eine Dauer von mindestens einer Woche hat.

2. Verfahren nach Anspruch 1, wobei, vor Trocknungsschritt f, das Festphasenmedium mit der Nukleinsäure gewaschen wird, um Kontaminanten zu entfernen, während die Nukleinsäure im Festphasenmedium zurückgehalten wird.

3. Verfahren nach Anspruch 1, wobei das getrocknete Festphasenmedium mit der Nukleinsäure in Schritt g im Wesentlichen auf einer Temperatur von 5 °C bis 40 °C gehalten wird.

4. Verfahren nach Anspruch 1, wobei, vor Elutionsschritt h, das getrocknete Festphasenmedium mit der Nukleinsäure gewaschen wird, um Kontaminanten zu entfernen, während die Nukleinsäure im Festphasenmedium zurückgehalten wird.

5. Verfahren nach Anspruch 1, wobei die Lagerung der Nukleinsäure in Schritt g eine Dauer von mindestens einem Monat hat.

6. Verfahren nach Anspruch 1, wobei die Lagerung der Nukleinsäure in Schritt g eine Dauer von mindestens drei Monaten hat.

7. Verfahren nach Anspruch 1, wobei die Lagerung der Nukleinsäure in Schritt g eine Dauer von mindestens fünf Monaten hat.

8. Verfahren nach Anspruch 1, wobei das Festphasenmedium einen Filter umfasst, der eine Mehrzahl von Fasern umfasst.

9. Verfahren nach Anspruch 8, wobei der Filter eine im Wesentlichen ungeordnete Struktur aufweist.

10. Verfahren nach Anspruch 8, wobei die Faserdurchmesser im Bereich von 1 µm bis 10 µm liegen.

11. Verfahren nach Anspruch 8, wobei der Filter eine oder mehrere Poren mit einer Porengröße von etwa 0,2 µm bis etwa 2,7 µm umfasst.

12. Verfahren nach Anspruch 1, wobei das Festphasenmedium umfasst:
a. ein Glas- oder Silica-basiertes Festphasenmedium;
b. ein Kunststoff-basiertes Festphasenmedium; oder
c. ein Cellulose-basiertes Festphasenmedium.

13. Verfahren nach Anspruch 1, wobei das Festphasenmedium aus einem der Folgenden gewählt ist: Glas, Glasfaser, Glasmikrofaser, Kieselsäure, Kieselgel, Siliciumoxid, Cellulose, Nitrocellulose, Carboxymethylcellulose, Polyester, Polyamid, Kohlenhydratpolymeren, Polypropylen, Polytetrafluorethylen, Polyvinylidenfluorid, Wolle oder poröser Keramik.

14. Verfahren nach Anspruch 1, wobei das Tensid oder Detergens aus Schritt d ein anionisches Tensid oder Detergens umfasst.

15. Verfahren nach Anspruch 14, wobei das anionische Tensid oder Detergens Natriumdodecylsulfat umfasst.

16. Verfahren nach Anspruch 15, wobei die Konzentration des Natriumdodecylsulfats zwischen etwa 0,5% und etwa 5% Gewicht/Volumen liegt.

17. Verfahren nach Anspruch 14, wobei die Lösung aus Schritt d weiterhin umfasst:
ii. eine schwache Base, und
iii. einen Chelatbildner.

18. Verfahren nach Anspruch 17, wobei die Lösung aus Schritt d weiterhin umfasst:
iv. Harnsäure oder ein Salz der Harnsäure.

19. Verfahren nach Anspruch 1, wobei das zelluläre Retentat kondensiertes Material aus dem Nukleus umfasst.

20. Verfahren nach Anspruch 1, wobei das zelluläre Retentat intakte ganze Zellen umfasst und wobei Schritt e umfasst:
i. Aufbrechen der vom Festphasenmedium zurückgehaltenen intakten ganzen Zellen so, dass kondensiertes Material aus dem Nukleus, zurückgehalten vom Medium, verbleibt; und
ii. Lysieren des kondensierten Materials aus dem Nukleus, um das Zelllysat zu bilden, das die Nukleinsäure enthält.

21. Verfahren nach Anspruch 1, wobei die Zusammensetzung und Dimensionen des Festphasenmediums so gewählt sind, dass die Nukleinsäure vom Medium in Schritt e im Wesentlichen durch nicht ionische Wechselwirkungen zurückgehalten wird.

22. Verfahren nach Anspruch 21, wobei die nicht ionischen Wechselwirkungen Dipol-Dipol-Wechselwirkungen, Dipol-induzierte Dipol-Wechselwirkungen, Dispersionskräfte oder Wasserstoffbrückenbindung umfassen.

23. Verfahren nach Anspruch 1, wobei der Zurückhalteschritt e weiterhin definiert ist als die Bewegung der Nukleinsäure durch das Festphasenmedium physikalisch verzögernd.

24. Verfahren nach Anspruch 1, wobei das Festphasenmedium befähigt ist, die Zellen und die Nukleinsäure bei Nichtvorhandensein eines chaotropen Mittels zurückzuhalten.

25. Verfahren nach Anspruch 1, wobei Schritt b weiterhin das Konzentrieren der Zellen im Festphasenmedium umfasst.

26. Verfahren nach Anspruch 1, wobei die Nukleinsäure vor Elutionsschritt h auf eine erhöhte Temperatur von 65 °C bis 125 °C erhitzt wird.

27. Verfahren nach Anspruch 1, wobei die Nukleinsäure vor Elutionsschritt h auf eine erhöhte Temperatur von 80 °C bis 95 °C erhitzt wird.

28. Verfahren nach Anspruch 1, wobei die Zellen aus jener Gruppe gewählt sind, die besteht aus weißen Blutkörperchen, Epithelzellen, bukkalen Zellen, Gewebekulturzellen, Sperma, Vaginalzellen, Harntraktzellen, Pflanzenzellen, bakteriellen Zellen und kolorektalen Zellen.

29. Verfahren nach Anspruch 1, wobei die Zellen weiße Blutkörperchen sind und das Verfahren weiterhin das Aufbringen von Vollblut auf das Festphasenmedium und das Erhalten des Zelllysats aus den weißen Blutkörperchen umfasst.

30. Verfahren nach Anspruch 29, das weiterhin das Lysieren der roten Blutkörperchen aus dem Vollblut umfasst.

31. Verfahren nach Anspruch 29 oder Anspruch 30, das weiterhin das Waschen des Festphasenmediums umfasst, um Kontaminationen zu entfernen.

32. Verfahren nach Anspruch 1, wobei die Probe Blutzellen umfasst und die Dimensionen des Festphasenmediums so gewählt sind, dass die Mehrheit der in Schritt c zurückgehaltenen Zellen weiße Blutkörperchen umfasst.

33. Verfahren nach Anspruch 1, wobei die Nukleinsäure DNA oder RNA umfasst.

34. Verfahren nach Anspruch 1, wobei die Nukleinsäure genomische DNA umfasst.

35. Verfahren zur Isolierung und Lagerung von Nukleinsäure, umfassend:
a. Bereitstellen eines Festphasenmediums;
b. Aufbringen einer Probe, die Nukleinsäure enthaltende Zellen umfasst, auf das Festphasenmedium, und Konzentrieren der Zellen im Festphasenmedium;
c. Zurückhalten der konzentrierten Zellen mit dem Festphasenmedium als konzentriertes zelluläres Retentat, und Entfernen von Kontaminanten;
d. Inkontaktbringen des konzentrierten zellulären Retentats mit einer Lösung, die umfasst:
i. eine schwache Base;
ii. einen Chelatbildner; und
iii. ein anionisches Tensid oder Detergens;
e. Lysieren des konzentrierten zellulären Retentats, um ein Zelllysat zu bilden, bei gleichzeitigem Zurückhalten des Zelllysats im Festphasenmedium, wobei das Zelllysat die Nukleinsäure umfasst;
f. Trocknen des Festphasenmediums mit dem die Nukleinsäure umfassenden Zelllysat;
g. Lagern des getrockneten Festphasenmediums mit der Nukleinsäure mindestens eine Woche lang; und
h. Eluieren der Nukleinsäure aus dem Festphasenmedium.

36. Verfahren zur Isolierung und Lagerung von DNA, umfassend:
a. Bereitstellen eines Festphasenmediums, wobei das Festphasenmedium einen Filter umfasst, der eine Mehrzahl von Fasern umfasst, wobei die Fasern umfassen:
i. Glas- oder Silica-basierte Fasern;
ii. Kunststoff-basierte Fasern; oder
iii. Nitrocellulose- oder Cellulose-basierte Fasern;
b. Aufbringen einer Probe, die DNA enthaltende Zellen umfasst, auf das Festphasenmedium, und Konzentrieren der Zellen im Festphasenmedium;
c. Zurückhalten der konzentrierten Zellen mit dem Festphasenmedium als konzentriertes zelluläres Retentat, und Entfernen von Kontaminanten;
d. Inkontaktbringen des konzentrierten zellulären Retentats mit einer Lösung, die umfasst:
i. eine schwache Base;
ii. einen Chelatbildner; und
iii. ein anionisches Tensid oder Detergens;
e. Lysieren des konzentrierten zellulären Retentats, um ein Zelllysat zu bilden, bei gleichzeitigem Zurückhalten des Zelllysats im Festphasenmedium, wobei das Zelllysat DNA enthält;
f. Trocknen des Festphasenmediums mit dem die DNA umfassenden Zelllysat;
g. Lagern des getrockneten Festphasenmediums mit der DNA bei einer Temperatur von 5 °C bis 40 °C mindestens eine Woche lang;
h. Erhitzen der DNA mit dem Festphasenmedium auf eine erhöhte Temperatur von 65 °C bis 125 °C; und
i. Eluieren der DNA aus dem Festphasenmedium.

## Revendications

1. Procédé d'isolement et de stockage d'acide nucléique, comprenant :
a. la préparation d'un milieu en phase solide ;
b. l'application d'un échantillon comprenant des cellules contenant de l'acide nucléique sur un milieu en phase solide ;
c. la retenue des cellules avec le milieu en phase solide sous forme d'un rétentat cellulaire et l'élimination des contaminants ;
d. la mise en contact du rétentat cellulaire avec une solution comprenant un surfactant ou un détergent ;
e. la lyse du rétentat cellulaire afin de former un lysat cellulaire tout en retenant le lysat cellulaire dans le milieu en phase solide, le lysat cellulaire comprenant l'acide nucléique ;
f. le séchage du milieu en phase solide avec le lysat cellulaire comprenant l'acide nucléique ;
g. le stockage du milieu en phase solide séché avec l'acide nucléique ; et
h. l'élution de l'acide nucléique à partir du milieu solide ;
dans lequel le stockage de l'acide nucléique à l'étape g présente une durée d'au moins une semaine.

2. Procédé selon la revendication 1, dans lequel, avant l'étape de séchage f, le milieu en phase solide avec l'acide nucléique est lavé afin d'éliminer les contaminants pendant que l'acide nucléique est retenu dans le milieu en phase solide.

3. Procédé selon la revendication 1, dans lequel le milieu en phase solide séché avec l'acide nucléique à l'étape g est conservé à une température sensiblement comprise entre 5°C et 40°C.

4. Procédé selon la revendication 1, dans lequel, avant l'étape d'élution h, le milieu en phase solide séché avec l'acide nucléique est lavé afin d'éliminer les contaminants pendant que l'acide nucléique est retenu dans le milieu en phase solide.

5. Procédé selon la revendication 1, dans lequel le stockage de l'acide nucléique à l'étape g présente une durée d'au moins un mois.

6. Procédé selon la revendication 1, dans lequel le stockage de l'acide nucléique à l'étape g présente une durée d'au moins trois mois.

7. Procédé selon la revendication 1, dans lequel le stockage de l'acide nucléique à l'étape g présente une durée d'au moins cinq mois.

8. Procédé selon la revendication 1, dans lequel le milieu en phase solide comprend un filtre comprenant une pluralité de fibres.

9. Procédé selon la revendication 8, dans lequel le filtre présente une structure sensiblement désordonnée.

10. Procédé selon la revendication 8, dans lequel les diamètres de fibre sont dans la plage de 1 µm à 10 µm.

11. Procédé selon la revendication 8, dans lequel le filtre comprend un ou plusieurs pores présentant un diamètre de pore compris entre 0,2 µm environ à 2,7 µm environ.

12. Procédé selon la revendication 1, dans lequel le milieu en phase solide comprend :
a. un milieu en phase solide à base de verre ou de silice ;
b. un milieu en phase solide à base de matière plastique ; ou
c. un milieu en phase solide à base de cellulose.

13. Procédé selon la revendication 1, dans lequel le milieu en phase solide est sélectionné à partir de l'un des éléments suivants : du verre, des fibres de verre, des microfibres de verre, de la silice, du gel de silice, de l'oxyde de silice, de la cellulose, de la nitrocellulose, de la carboxyméthylcellulose, du polyester, du polyamide, des polymères d'hydrate de carbone, du polypropylène, du polytétrafluroréthylène, du fluorure de polyvinylidène, de la laine, ou des céramiques poreuses.

14. Procédé selon la revendication 1, dans lequel le surfactant ou détergent de l'étape d comprend un surfactant ou détergent anionique.

15. Procédé selon la revendication 14, dans lequel le surfactant ou détergent anionique comprend du sulfate dodécylique de sodium.

16. Procédé selon la revendication 15, dans lequel la concentration du sulfate dodécylique de sodium est comprise entre 0,5 % environ et 5 % en poids/volume environ.

17. Procédé selon la revendication 14, dans lequel la solution de l'étape d comprend, en outre :
ii. une base faible, et
iii. un agent de chélation.

18. Procédé selon la revendication 17, dans lequel la solution de l'étape d comprend, en outre :
iv. de l'acide urique ou un sel d'urate.

19. Procédé selon la revendication 1, dans lequel le rétentat cellulaire comprend un matériau condensé à partir du noyau.

20. Procédé selon la revendication 1, dans lequel le rétentat cellulaire comprend des cellules entières intactes et dans lequel l'étape e comprend :
i. la rupture des cellules entières intactes retenues par le milieu en phase solide afin de laisser le matériau condensé à partir du noyau retenu par le milieu ; et
ii. la lyse du matériau condensé à partir du noyau afin de former le lysat cellulaire contenant l'acide nucléique.

21. Procédé selon la revendication 1, dans lequel la composition et les dimensions du milieu en phase solide sont sélectionnées de telle sorte que l'acide nucléique soit retenu par le milieu à l'étape e sensiblement par des interactions non ioniques.

22. Procédé selon la revendication 21, dans lequel les interactions non ioniques comprennent les interactions dipôle-dipôle, les interactions dipôle dipôle induit, les forces de dispersion, ou par liaison hydrogène.

23. Procédé selon la revendication 1, dans lequel l'étape de retenue e est, en outre, définie comme retardant physiquement le mouvement de l'acide nucléique à travers le milieu en phase solide.

24. Procédé selon la revendication 1, dans lequel le milieu en phase solide peut retenir les cellules et l'acide nucléique en l'absence d'un agent chaotropique.

25. Procédé selon la revendication 1, dans lequel l'étape b comprend, en outre, la concentration des cellules dans le milieu en phase solide.

26. Procédé selon la revendication 1, dans lequel l'acide nucléique est chauffé à une température élevée de 65°C à 125°C avant l'étape d'élution h.

27. Procédé selon la revendication 1, dans lequel l'acide nucléique est chauffé à une température élevée de 80°C à 95°C avant l'étape d'élution h.

28. Procédé selon la revendication 1, dans lequel les cellules sont sélectionnées à partir du groupe constitué par des globules blancs , des cellules épithéliales, des cellules buccales, des cellules de culture de tissu, du sperme, des cellules vaginales, des cellules de conduit urinaire, des cellules de plante, des cellules bactériennes, et des cellules colorectales.

29. Procédé selon la revendication 1, dans lequel les cellules sont des globules blancs et le procédé comprend, en outre, l'application de sang entier sur le milieu en phase solide, et l'obtention du lysat cellulaire à partir des globules blancs.

30. Procédé selon la revendication 29, qui comprend, en outre, la lyse des globules rouges du sang entier.

31. Procédé selon la revendication 29 ou 30, qui comprend, en outre, la lavage du milieu en phase solide de manière à éliminer les contaminations.

32. Procédé selon la revendication 1, dans lequel l'échantillon comprend des cellules sanguines et les dimensions du milieu en phase solide sont sélectionnées de telle sorte que la majorité des cellules retenues à l'étape c comprennent des globules blancs.

33. Procédé selon la revendication 1, dans lequel l'acide nucléique comprend de l'ADN ou de l'ARN.

34. Procédé selon la revendication 1, dans lequel l'acide nucléique comprend de l'ADN génomique.

35. Procédé d'isolement et de stockage d'acide nucléique, comprenant :
a. la préparation d'un milieu en phase solide ;
b. l'application d'un échantillon comprenant des cellules contenant de l'acide nucléique sur le milieu en phase solide et la concentration les cellules dans le milieu en phase solide ;
c. la retenue des cellules concentrées avec le milieu en phase solide sous forme d'un rétentat cellulaire concentré et l'élimination des contaminants ;
d. la mise en contact du rétentat cellulaire concentré avec une solution comprenant :
i. une base faible ;
ii. un agent de chélation ; et
iii. un surfactant ou détergent anionique ;
e. la lyse du rétentat cellulaire concentré afin de former un lysat cellulaire tout en retenant le lysat cellulaire dans le milieu en phase solide, le lysat cellulaire comprenant l'acide nucléique ;
f. le séchage du milieu en phase solide avec le lysat cellulaire comprenant l'acide nucléique ;
g. le stockage du milieu en phase solide séché avec l'acide nucléique pendant au moins une semaine ; et
h. l'élution de l'acide nucléique du milieu en phase solide.

36. Procédé d'isolement et de stockage d'ADN ; comprenant :
a. la préparation d'un milieu en phase solide, dans lequel le milieu en phase solide comprend un filtre comprenant une pluralité de fibres, dans lequel les fibres comprennent :
i. des fibres à base de verre ou de silice ;
ii. des fibres à base de matière plastique ; ou
iii. des fibres à base de cellulose ou de nitrocellulose ;
b. l'application d'un échantillon comprenant des cellules contenant de l'ADN sur le milieu en phase solide et la concentration des cellules dans le milieu en phase solide ;
c. la retenue des cellules concentrées avec le milieu en phase solide sous forme d'un rétentat cellulaire concentré et l'élimination des contaminants ;
d. la mise en contact du rétentat cellulaire concentré avec une solution comprenant :
i. une base faible ;
ii. un agent de chélation ; et
iii. un surfactant ou détergent anionique ;
e. la lyse du rétentat cellulaire concentré afin de former un lysat cellulaire tout en retenant le lysat cellulaire dans le milieu en phase solide, le lysat cellulaire contenant l'ADN ;
f. le séchage du milieu en phase solide avec le lysat cellulaire comprenant l'ADN ;
g. le stockage du milieu en phase solide séché avec l'ADN à une température de 5°C à 40°C pendant au moins une semaine ;
h. le chauffage de l'ADN avec le milieu en phase solide à une température élevée de 65 °C à 125 °C ; et
i. l'élution de l'ADN du milieu en phase solide.
